# EUROPEAN PATENT APPLICATION

(11) **EP 4 710 980 A1**
(43) Date of publication of application: **18.03.2026**
(21) Application number: 23937374.9
(22) Date of filing: 12.05.2023
(51) Int. Cl.: A61M 25/00

(54) **MEDICAL DEVICE**

(71) Applicant: ASAHI INTECC CO., LTD., Seto-shi, Aichi, 489-0071 (JP)
(72) Inventor: SHIMIZU Hirotomo, Seto-shi, Aichi 489-0071 (JP); HASE Yukiko, Seto-shi, Aichi 489-0071 (JP); YAMADA Kiyotaka, Seto-shi, Aichi 489-0071 (JP)
(74) Representative: Winter, Brandl - Partnerschaft mbB
(86) International application number: PCT/JP2023/017873
(87) International publication number: WO 2024/236627

(57) **Abstract**

A medical device includes a distal tip, a first reinforcing layer, and a resin layer. The distal tip contains resin material and radiopaque material. The first reinforcing layer is connected to a proximal end of the distal tip. The resin layer is arranged in a radially outward side of the distal tip, has a distal end located further in a distal end side than the proximal end of the distal tip, and having a material property different from the material property of the distal tip.

## Description

### [Technical Field]

The technology disclosed herein relates to a medical device.

### [Background Art]

Treatment using a catheter is widely performed for a case where a constricted part or an occluded part (hereinafter, collectively referred to as a "constricted part") occurs in a body lumen (digestive organs such as bile duct, gallbladder, pancreas, esophagus, duodenum, small intestine, or large intestine, blood vessel, ureter, trachea, or the like), for example. A catheter has a configuration in which a distal tip formed of resin material is bonded to a distal end of a shaft with a reinforcing layer composed of a coil body, for example. With such configuration of the catheter, the shaft having relatively high stiffness ensures operability while the relatively flexible distal tip prevents damage to the luminal wall of the body.

For example, when the catheter is inserted into a lumen with a wide curve in the body, or when the catheter is operated with the distal tip caught in a constricted part, there is a risk of breakage of the distal tip or breakage from the shaft. A conventional configuration has been proposed, in which two reinforcing layers are provided in the shaft and a proximal end portion of the distal tip is extended to between the two reinforcing layers of the shaft in order to suppress breakage of the distal tip (see, for example, Patent Literature 1).

### [Citation List]

### [Patent Literature]

[Patent Literature 1] WO 2018/042596

### [Summary of Invention]

### [Technical Problem]

The conventional configuration described above does not sufficiently suppress breakage of the distal tip. In particular, when radiopaque material is included in the distal tip to ensure visibility of the distal tip under radiation without a marker formed of radiopaque material, the flexibility (extensibility) of the distal tip is reduced, and a technology to more effectively suppress breakage of the distal tip is desired. This problem is not limited to catheters, but is common to medical devices with a distal tip and a reinforcing layer connected to a proximal end of the distal tip.

A technology capable of solving the above problems is disclosed herein.

### [Solution to Problem]

The technology disclosed herein can be implemented as the following aspects, for example.

(1) A medical device disclosed herein includes a distal tip, a reinforcing layer, and a resin layer. The distal tip contains resin material and radiopaque material. The reinforcing layer is connected to a proximal end of the distal tip. The resin layer is arranged in a radially outward side of the distal tip, has a distal end located further in a distal end side than the proximal end of the distal tip, and having a predetermined material property different from the predetermined material property of the distal tip.

In this medical device, since the distal tip contains resin material and radiopaque material, the presence of the resin material ensures flexibility (extensibility) of the distal tip, while the presence of the radiopaque material ensures visibility of the distal tip under radiation without a separate marker or even with a thin marker. In this medical device, the distal end of the resin layer having a different material property from a material property of the distal tip is located further on the distal end side of the proximal end of the distal tip, so that the presence of the resin layer can reinforce the proximal end of the distal tip and prevents breakage of the distal tip. Moreover, the presence of the resin layer can mitigate a stiffness gap at a connection position between the distal tip and the reinforcing layer in the medical device.

(2) The medical device described above may have such a configuration that the reinforcing layer is a first reinforcing layer, the medical device further includes a second reinforcing layer having a distal end located further in the distal end side than a proximal end of the distal tip, and the distal end of the resin layer is located further in the distal end side than a distal end of the second reinforcing layer. According to this configuration, a resin layer with high extensibility can be arranged at a position of the distal end of the second reinforcing layer that is prone to breaking due to a relatively large stiffness gap in the distal tip, and breakage of the distal tip can be more effectively suppressed.

(3) The medical device described above may have such a configuration that the medical device further includes an inner layer arranged in a radially inward side of the second reinforcing layer and has a distal end located further in the distal end side than the distal end of the second reinforcing layer. According to this configuration, the presence of the inner layer can effectively mitigate the stiffness gap at the position of the distal end of the second reinforcing layer, and breakage of the distal tip can be more effectively suppressed.

(4) The medical device described above may have such a configuration that the distal tip includes a taper that tapers toward a distal end and a straight portion arranged in a proximal end of the taper, and the distal end of the second reinforcing layer is located further in a proximal end side than a boundary of the taper and the straight portion. According to this configuration, appropriate gradually changed stiffness can be achieved.

(5) The medical device described above may have such a configuration that the predetermined material property is a content ratio of the radiopaque material, and the content ratio of the radiopaque material in the resin layer is lower than a content ratio of the radiopaque material in the distal tip. According to this configuration, the distal end of the resin layer is located further in the distal end side than the proximal end of the distal tip. The resin layer has high flexibility (extensibility) since the resin layer contains a lower percentage of the radiopaque material (that is, a higher percentage of the resin material) compared to the distal tip. Therefore, the presence of the resin layer with high extensibility can suppress the breakage of the distal tip. Moreover, the presence of the resin layer having high flexibility can mitigate a stiffness gap at a connection position between the distal tip and the reinforcing layer in the medical device.

(6) The medical device described above may have such a configuration that the resin layer does not contain radiopaque material. According to this configuration, the flexibility (extensibility) of the resin layer can be effectively increased and the breakage of the distal tip can be effectively suppressed.

(7) The medical device described above may have such a configuration that the resin layer and the distal tip contain the same type of resin material. According to this configuration, an interfacial bonding force between the resin layer and the distal tip can be made stronger, and breakage of the distal tip can be effectively suppressed.

The technology disclosed herein can be achieved in various forms, for example, in the form of a medical device such as a catheter, a medical system including a medical device, and a method of manufacturing a medical device or a medical system.

### [Brief Description of Drawings]

FIG. 1 is a diagram that shows a configuration of a side surface of a catheter in a first embodiment.
FIG. 2 is a diagram that shows a configuration of a vertical cross section (YZ section) of a distal end portion of the catheter in the first embodiment.
FIG. 3 is a diagram that shows a configuration of a vertical cross section of a distal end portion of a catheter in a second embodiment.
FIG. 4 is a diagram that shows a configuration of a vertical cross section of a distal end portion of a catheter in a third embodiment.
FIG. 5 is a diagram that shows a configuration of a vertical cross section of a distal end portion of a catheter in a fourth embodiment.

### [Description of Embodiment]

### A. First Embodiment:

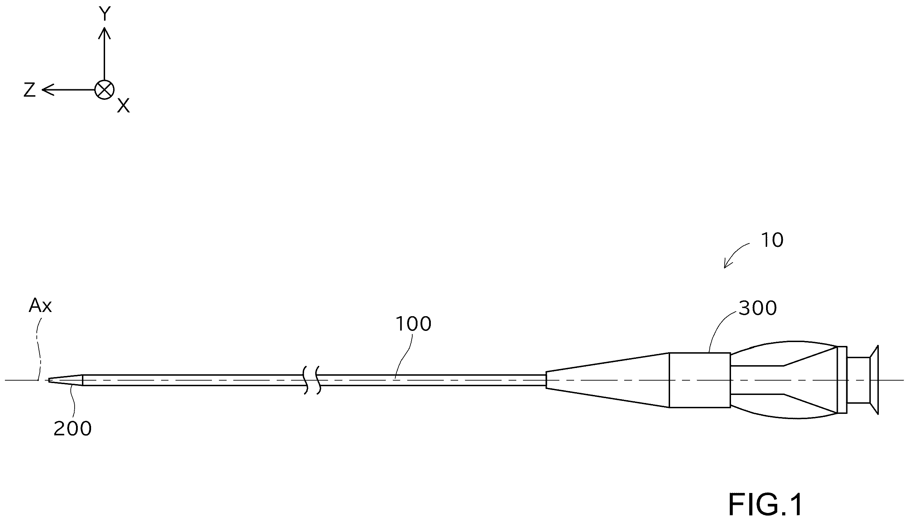

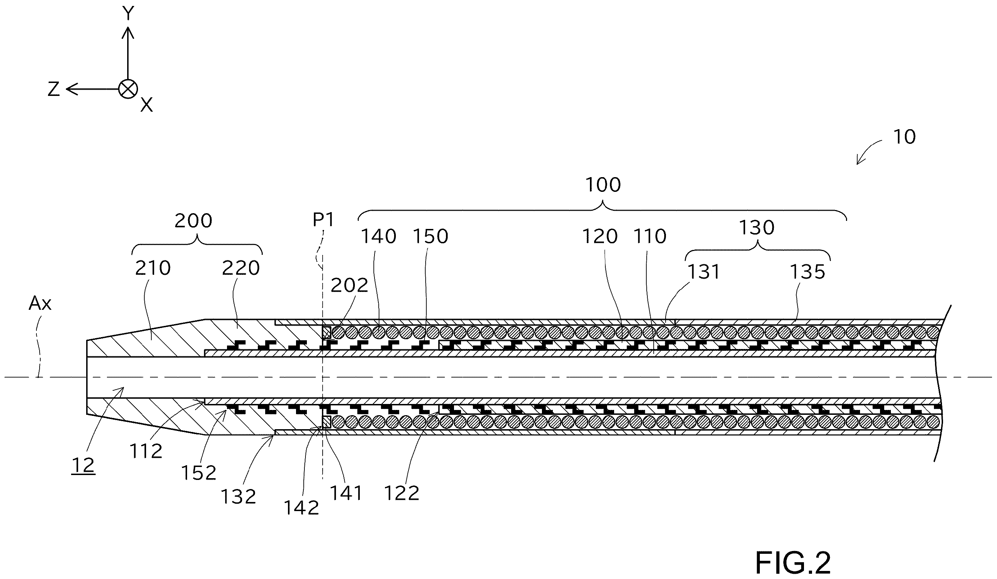

FIG. 1 is a diagram that shows a configuration of a side surface of a catheter 10 in a first embodiment, and FIG. 2 is a diagram that shows a configuration of a vertical cross section (YZ cross section) of a distal end portion of the catheter 10 in the first embodiment. In each drawing, a portion of the catheter 10 is omitted. In each drawing, the Z axis positive direction side is a distal end side (distal side) to be inserted into a body, and the Z axis negative direction side is a proximal end side (near side) to be operated by a professional such as a doctor. In each drawing, a center axis Ax of a shaft 100 of the catheter 10, described below, is shown parallel to the Z axis, but the shaft 100 is configured to be bendable. In the description, regarding the catheter 10 and each configuration member thereof, an end on the distal end side is referred to as "distal end", the distal end and a portion in the vicinity thereof are referred to as "distal end portion", an end on the proximal end side is referred to as "proximal end", and the proximal end and a portion in the vicinity thereof are referred to as "proximal end portion".

The catheter 10 is a medical device. The catheter 10 is inserted into a blood vessel. The full length of the catheter 10 ranges from about 1000 mm to 2000 mm, for example.

The catheter 10 includes a shaft 100, a distal tip 200, and a connector 300. At least a part of the catheter 10 may have a hydrophilic coating.

The connector 300 is a member connected to the proximal end portion of the shaft 100 and constituting the most proximal end part of the catheter 10. The connector 300 may have a port for connection to a syringe or the like. The connector 300 is formed of a resin, for example.

The shaft 100 is a long and hollow member. A contour of a transverse cross section (XY cross section) of the shaft 100 at each position can take any shape, for example, a substantially circular shape or a substantially oval shape. The outer diameter of the shaft 100 ranges from about 0.2 mm to 1.5 mm, for example. The outer diameter of shaft 100 may be substantially constant from the proximal end to the distal end or may vary along the longitudinal direction, for example, gradually decreasing in diameter from the proximal end to the distal end.

As shown in FIG. 2, the shaft 100 includes a first resin layer (inner layer) 110, a second resin layer (middle layer) 120, a third resin layer (outer layer) 130, a first reinforcing layer 140, and a second reinforcing layer 150.

The first resin layer 110 is a cylindrical body that defines the inner peripheral surface of a lumen 12 into which a guidewire is inserted, for example. The second resin layer 120 is a cylindrical body arranged in the radially outward side of the first resin layer 110. The third resin layer 130 is a cylindrical body arranged in the radially outward side of the second resin layer 120. In the present embodiment, the third resin layer 130 includes a distal-end-side third resin layer 131 that constitutes the distal end portion of the third resin layer 130 and a proximal-end-side third resin layer 135 located at the proximal end side of the distal-end-side third resin layer 131. The boundary between the distal-end-side third resin layer 131 and the proximal-end-side third resin layer 135 is located further in the proximal end side than the proximal end 202 of the distal tip 200. The distal-end-side third resin layer 131 is an example of a resin layer.

The first resin layer 110 is formed of, for example, polytetrafluoroethylene (PTFE). The second resin layer 120 is formed of, for example, polyamide. In the third resin layer 130, the distal-end-side third resin layer 131 is formed of a relatively flexible resin material such as urethane, for example, and the proximal-end-side third resin layer 135 is formed of polyamide, for example. The distal-end-side third resin layer 131 does not contain radiopaque material. In this description, "containing no radiopaque material" means substantially containing no radiopaque material, and includes a mode in which radiopaque material is contained as an impurity.

The first reinforcing layer 140 is a member to increase the stiffness of the shaft 100 and is arranged between the second resin layer 120 and the third resin layer 130. In the present embodiment, the first reinforcing layer 140 includes a coil body with strands of wire spirally wound around it. At the distal end of the first reinforcing layer 140, a weld 141 is formed to bring together the strands of the coil body. The weld 141 constitutes the distal end 142 of the first reinforcing layer 140. The first reinforcing layer 140 is formed of, for example, a metallic material such as stainless steel such as SUS304, tungsten, or Ni-Ti alloy, or resin material such as PEEK.

The second reinforcing layer 150 is a member to increase the stiffness of the shaft 100 and is arranged along the outer peripheral surface of the first resin layer 110. In the present embodiment, the portions of the second reinforcing layer 150, except for the distal end portion of the second reinforcing layer 150, are buried in the second resin layer 120. In the present embodiment, the second reinforcing layer 150 is composed of a cylindrical body (so-called braid) made of a plurality of strands woven into a mesh (mesh-like) pattern. The second reinforcing layer 150 is formed of, for example, a metallic material such as stainless steel such as SUS304, tungsten, or Ni-Ti alloy, or resin material such as PEEK.

The distal tip 200 is a hollow member that constitutes the most distal end portion of the catheter 10. The hollow portion of the distal tip 200 communicates with the hollow portion of the shaft 100 and defines the inner peripheral surface of the lumen 12 of the catheter 10. The distal tip 200 is formed of a relatively flexible resin material, such as urethane, for example. The distal tip 200 contains radiopaque material (for example, tungsten) at a predetermined content ratio. As described above, the distal-end-side third resin layer 131 does not contain radiopaque material, so the property of the distal-end-side third resin layer 131 and the property of the distal tip 200 are different from each other in terms of the content ratio of radiopaque material. The content ratio of radiopaque material is an example of a predetermined material property. It is preferable that the distal-end-side third resin layer 131 and the distal tip 200 contain the same type of resin material. It is further preferable that the distal-end-side third resin layer 131 and the distal tip 200 contain the same type and grade of resin material.

The distal tip 200 includes a taper 210 and a straight portion 220. The taper 210 constitutes the distal end portion of the distal tip 200. The taper 210 has a tapered shape in which the outer diameter gradually decreases toward the distal end. The straight portion 220 is arranged at the proximal end of the taper 210 and constitutes the proximal end portion of the distal tip 200. The straight portion 220 has a straight shape whose outer diameter is equal to or greater than the outer diameter of the proximal end of taper 210.

The distal end 142 of the first reinforcing layer 140 is connected to the proximal end 202 of the distal tip 200. In other words, the distal tip 200 and shaft 100 are connected to each other at the boundary position (hereinafter, referred to as "connection position P1") between the distal end 142 of the first reinforcing layer 140 and the proximal end 202 of the distal tip 200. However, the first resin layer 110 and the second reinforcing layer 150 constituting the shaft 100 extend beyond the proximal end 202 (connection position P1) of the distal tip 200 and further to the distal end side. In the present embodiment, the distal end 112 of the first resin layer 110 is located at the same position as or further in the distal end side than the distal end 152 of the second reinforcing layer 150. The distal end 122 of the second resin layer 120 is located further in the proximal end side than the proximal end 202 of the distal tip 200 (connection position P1).

The distal-end-side third resin layer 131 of the third resin layer 130 constituting the shaft 100 extends beyond the proximal end 202 (connection position P1) of the distal tip 200 and further to the distal end side. In other words, the distal-end-side third resin layer 131 extends in the direction of the center axis Ax so as to straddle the connection position P1 between the distal tip 200 and the shaft 100. In other words, the distal-end-side third resin layer 131 extends in the direction of the center axis Ax so as to straddle the connection position P1 between the distal tip 200 and the main part of the shaft 100. In the present embodiment, the distal end 132 of the distal-end-side third resin layer 131 is located further in the proximal end side than the distal end 152 of the second reinforcing layer 150. It is preferable that the distance from the connection position P1 along the direction of the center axis Ax to the distal end 132 of the distal-end-side third resin layer 131 is equal to or greater than 1/3 of the length of the straight portion 220 of the distal tip 200, and it is more preferable that the distance is equal to or greater than 2/3 of the length of the straight portion 220 of the distal tip 200.

The catheter 10 with such a configuration can be made, for example, in bonding the distal tip 200 to the shaft 100, by covering the outer peripheral surface of the proximal end portion of distal tip 200 by the distal end portion of the distal-end-side third resin layer 131 and then bonding the distal tip 200 to the shaft 100 by welding.

As described above, the catheter 10 of the present embodiment includes a distal tip 200, a first reinforcing layer 140, and a distal-end-side third resin layer 131. The distal tip 200 contains resin material and radiopaque material. The first reinforcing layer 140 is connected to the proximal end of the distal tip 200. The distal-end-side third resin layer 131 is arranged in the radially outward side of the distal tip 200. The distal end 132 of the distal-end-side third resin layer 131 is located further in the distal end side than the proximal end 202 of the distal tip 200. The content ratio of the radiopaque material of the distal-end-side third resin layer 131 is different from the content ratio of the radiopaque material of the distal tip 200. More specifically, the content ratio of the radiopaque material in the distal-end-side third resin layer 131 is lower than the content ratio of the radiopaque material in the distal tip 200.

As described above, in the catheter 10 of the present embodiment, since the distal tip 200 contains resin material and radiopaque material, the presence of the resin material ensures flexibility (extensibility) of the distal tip 200, while the presence of the radiopaque material ensures visibility of the distal tip 200 under radiation without a separate marker or even with a thin marker. In the catheter 10 of the present embodiment, the distal tip 132 of the distal-end-side third resin layer 131 is located further in the distal end side than the proximal end 202 of the distal tip 200. The distal-end-side third resin layer 131 has high flexibility (extensibility) since the distal-end-side third resin layer 131 contains a lower percentage of the radiopaque material (that is, a higher percentage of the resin material) compared to the distal tip 200, Therefore, the presence of the distal-end-side third resin layer 131 having high extensibility can reinforce the proximal end 202 of the distal tip 200 and prevent breakage of the distal tip 200. Moreover, the presence of the distal-end-side third resin layer 131 having high flexibility can mitigate the stiffness gap at the connection position P1 between the distal tip 200 and the first reinforcing layer 140 in the catheter 10.

In the catheter 10 of the present embodiment, the distal-end-side third resin layer 131 does not contain radiopaque material. Therefore, the flexibility (extensibility) of the distal-end-side third resin layer 131 can be effectively increased, and the breakage of the distal tip 200 can be effectively suppressed.

In the catheter 10 of the present embodiment, it is preferable that the distal-end-side third resin layer 131 and the distal tip 200 contain the same type of resin material. According to such a configuration, an interfacial bonding force between the distal-end-side third resin layer 131 and the distal tip 200 can be made stronger, and breakage of the distal tip 200 can be effectively suppressed.

An example of the catheter 10 having the configuration of the above-described embodiment was manufactured and a tensile test was performed. The result of the tensile test is shown in Table 1.

**[Table 1]**

| Breaking strength (N) | Example | Comparative example |
|---|---|---|
| Average value | 7.19 | 5.13 |
| Minimum value | 4.81 | 3.35 |
| Maximum value | 11.63 | 7.95 |
| Standard deviation | 1.27 | 1.38 |

Table 1 shows the breaking strength (average value, a minimum value, a maximum value, and a standard deviation) identified by the tensile test. The comparative example is in the mode where the distal end 132 of the distal-end-side third resin layer 131 is located at the position of the proximal end 202 of the distal tip 200. In other words, in the mode of the comparative example, the connection position P1 between the distal tip 200 and the shaft 100 is not covered by the distal-end-side third resin layer 131. The average value of the breaking strength of the catheter in the example was about 2 N greater than the average value of the breaking strength of the catheter in the comparative example. According to this result, it was confirmed that the catheter 10 having the configuration of the present embodiment can suppress breakage of the distal tip 200.

### B. Second Embodiment:

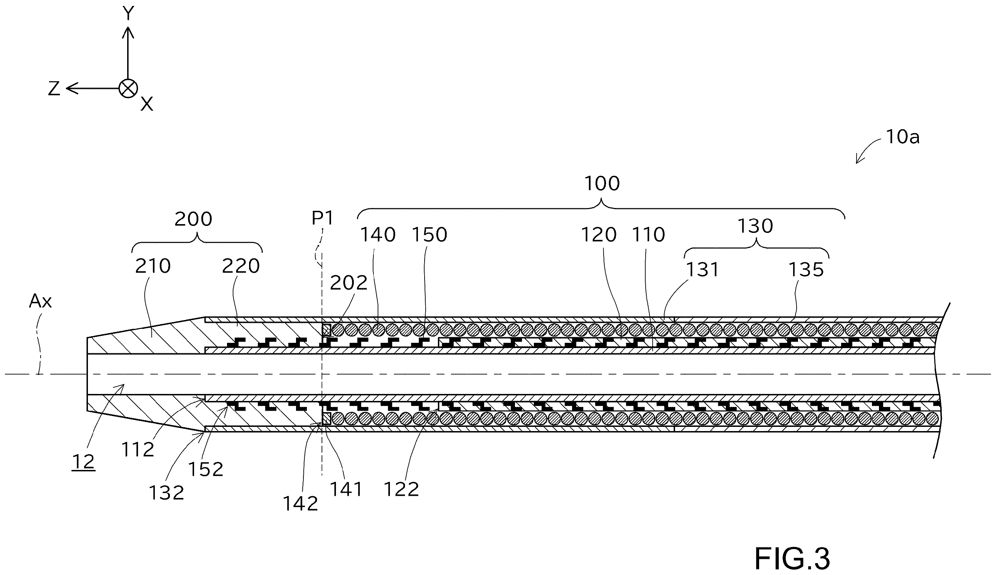

FIG. 3 is a diagram that shows a configuration of a vertical cross section of a distal end portion of a catheter 10a in a second embodiment. Hereinafter, the description of any component that is the same as that of the catheter 10 of the above-described first embodiment among the components of the catheter 10a of the second embodiment is omitted as appropriate by marking it with the same symbol.

The catheter 10a of the second embodiment is different from the catheter 10 of the first embodiment in the configuration of the distal-end-side third resin layer 131. Specifically, in the second embodiment, as in the first embodiment, the distal end 132 of the distal-end-side third resin layer 131 is located further in the distal end side than the proximal end 202 of the distal tip 200. In the second embodiment, unlike the first embodiment, the distal-end-side third resin layer 131 further extends to the distal end side, and the distal end 132 of the distal-end-side third resin layer 131 is located further in the distal end side than the distal end 152 of the second reinforcing layer 150. In other words, the distal-end-side third resin layer 131 extends in the direction of the center axis Ax so as to straddle the connection position P1 between the distal tip 200 and the shaft 100 and straddle the position of the distal end 152 of the second reinforcing layer 150.

As described above, in the catheter 10a of the second embodiment, the distal end 152 of the second reinforcing layer 150 is located further in the distal end side than the proximal end 202 of the distal tip 200. The distal end 132 of the distal-end-side third resin layer 131 is located further in the distal end side than the distal end 152 of the second reinforcing layer 150. Therefore, according to the catheter 10a of the second embodiment, the distal-end-side third resin layer 131 having high extensibility can be arranged at the position of the distal end 152 of the second reinforcing layer 150 that is prone to breaking due to the relatively large stiffness gap in the distal tip 200, and breakage of the distal tip 200 can be more effectively suppressed.

The catheter 10a of the second embodiment further includes a first resin layer 110 arranged in the radially inward side of the second reinforcing layer 150. The distal end 112 of the first resin layer 110 is located further in the distal end side than the distal end 152 of the second reinforcing layer 150. Therefore, according to the catheter 10a of the second embodiment, the presence of the first resin layer 110 can effectively mitigate the stiffness gap at the position of the distal end 152 of the second reinforcing layer 150, which can further effectively suppress the breakage of the distal tip 200.

In the second embodiment of the catheter 10a, the distal end 152 of the second reinforcing layer 150 is located further in the proximal end side than the boundary between the taper 210 and the straight portion 220 of the distal tip 200. When the distal end 152 of the second reinforcing layer 150 is located at the boundary described above, the stiffness gap at the position of the boundary becomes larger. When the distal end 152 of the second reinforcing layer 150 is positioned further in the distal end side than the boundary described above, a stiffness gap due to the presence or absence of the second reinforcing layer 150 is generated in the taper 210 that is a thin portion of the distal tip 200, and therefore, the stiffness gap becomes larger at the distal end 152 of the second reinforcing layer 150. In contrast, in the catheter 10a of the second embodiment, the distal end 152 of the second reinforcing layer 150 is located further in the proximal end side than the boundary described above, so that appropriate gradually changed stiffness can be achieved.

### C. Third Embodiment:

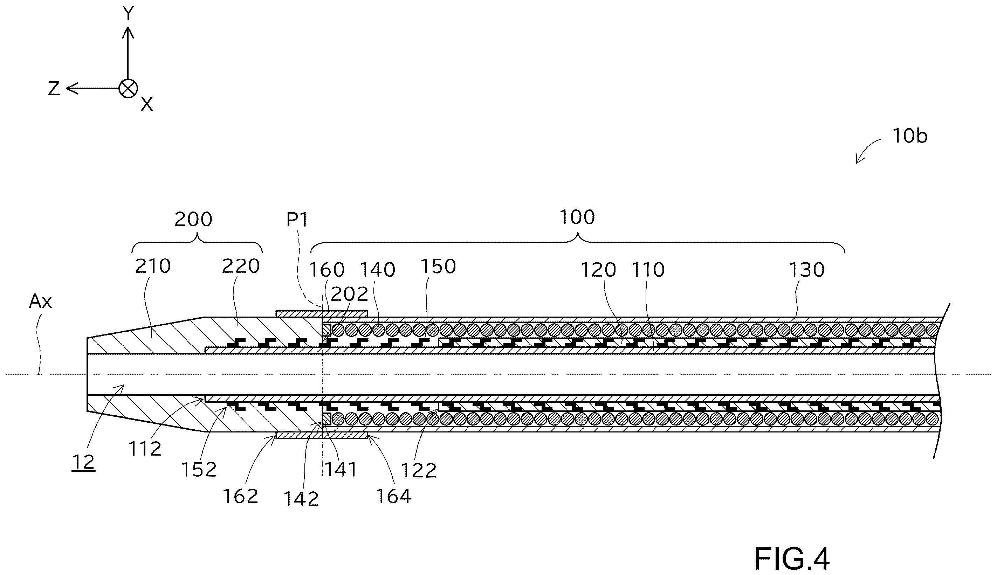

FIG. 4 is a diagram that shows a configuration of a vertical cross section of a distal end portion of a catheter 10b in a third embodiment. Hereinafter, the description of any component that is the same as that of the catheter 10 of the above-described first embodiment among the components of the catheter 10b of the third embodiment is omitted as appropriate by marking it with the same symbol.

The catheter 10b of the third embodiment is different from the catheter 10 of the first embodiment in the configuration of the third resin layer 130. Specifically, in the third embodiment, the third resin layer 130 is not divided into a distal-end-side third resin layer 131 and a proximal-end-side third resin layer 135, and is formed of a single resin material (for example, polyamide). The distal end of the third resin layer 130 is at substantially the same location as the proximal end 202 of the distal tip 200.

In the third embodiment, the shaft 100 further includes a fourth resin layer 160. The fourth resin layer 160 is formed, for example, of a relatively flexible urethane resin. The fourth resin layer 160 does not contain radiopaque material. The fourth resin layer 160 is arranged along the outer peripheral surface of the third resin layer 130 and the outer peripheral surface of the distal tip 200. The distal end 162 of the fourth resin layer 160 is located further in the distal end side than the proximal end 202 of the distal tip 200 and the proximal end 164 of the fourth resin layer 160 is located further in the proximal end side than the proximal end 202 of the distal tip 200. In other words, the fourth resin layer 160 extends in the direction of the center axis Ax so as to straddle the connection position P1 between the distal tip 200 and the shaft 100. The fourth resin layer 160 is an example of a resin layer.

As described above, the catheter 10b of the third embodiment includes the fourth resin layer 160 arranged in the radially outward side of the distal tip 200. The distal end 162 of the fourth resin layer 160 is located further in the distal end side than the proximal end 202 of the distal tip 200. The content ratio of the radiopaque material of the fourth resin layer 160 is different from the content ratio of the radiopaque material of the distal tip 200. More specifically, the content ratio of the radiopaque material in the fourth resin layer 160 is lower than the content ratio of the radiopaque material in the distal tip 200. In other words, the distal end 162 of the fourth resin layer 160 having high flexibility (extensibility) due to its lower content ratio of radiopaque material than the content ratio of radiopaque material of the distal tip 200 is located further in the distal end side than the proximal end 202 of the distal tip 200. Therefore, the presence of the fourth resin layer 160 having high extensibility can reinforce the proximal end 202 of the distal tip 200 and prevent breakage of the distal tip 200. Moreover, the presence of the fourth resin layer 160 having high flexibility can mitigate the stiffness gap at the connection position P1 between the distal tip 200 and the first reinforcing layer 140 in the catheter 10b.

### D. Fourth Embodiment

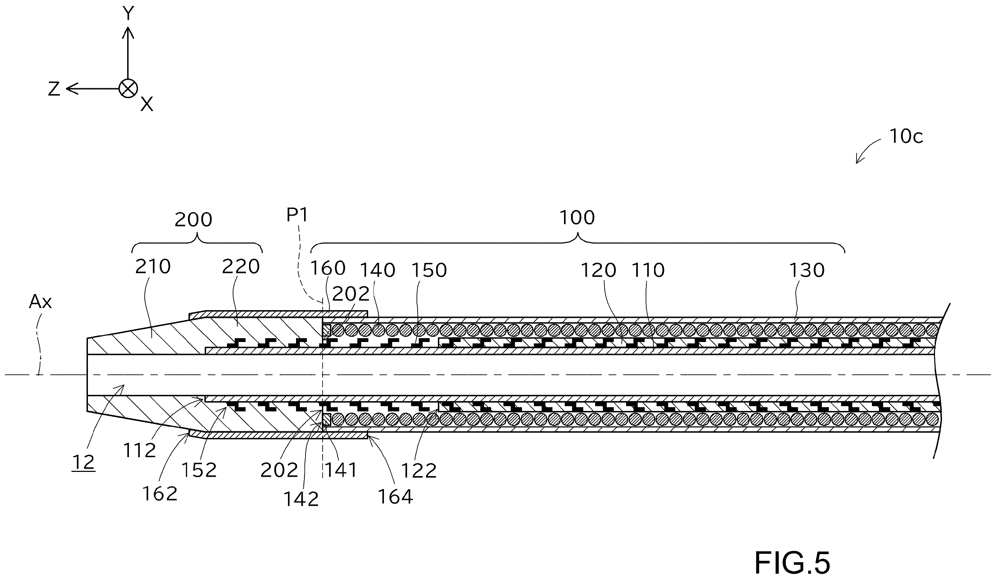

FIG. 5 is a diagram that shows a configuration of a vertical cross section of a distal end portion of a catheter 10c in a fourth embodiment. Hereinafter, the description of any component that is the same as that of the catheter 10b of the above-described third embodiment among the components of the catheter 10c of the fourth embodiment is omitted as appropriate by marking it with the same symbol.

The catheter 10c of the fourth embodiment is different from the catheter 10b of the third embodiment in the configuration of the fourth resin layer 160. Specifically, in the fourth embodiment, as in the third embodiment, the distal end 162 of the fourth resin layer 160 is located further in the distal end side than the proximal end 202 of the distal tip 200. In the fourth embodiment, unlike the third embodiment, the fourth resin layer 160 further extends to the distal end side, and the distal end 162 of the fourth resin layer 160 is located further in the distal end side than the distal end 152 of the second reinforcing layer 150. In other words, the fourth resin layer 160 extends in the direction of the center axis Ax so as to straddle the connection position P1 between the distal tip 200 and the shaft 100 and straddle the position of the distal end 152 of the second reinforcing layer 150.

As described above, in the catheter 10c of the fourth embodiment, the distal end 162 of the fourth resin layer 160 is located further in the distal end side than the distal end 152 of the second reinforcing layer 150. Therefore, according to the catheter 10c of the fourth embodiment, the fourth resin layer 160 having high extensibility can be arranged at the position of the distal end 152 of the second reinforcing layer 150 that is prone to breaking due to the relatively large stiffness gap in the distal tip 200, and breakage of the distal tip 200 can be more effectively suppressed.

### E. Modification Example

The technology disclosed herein is not limited to the above embodiments, and can be modified in various forms without departing from the gist thereof. For example, the following modification examples are also possible.

The configurations of the catheter 10 in the above embodiments are only examples and can be modified in various forms. For example, in the above embodiments, the shaft 100 has two reinforcing layers, the first reinforcing layer 140 and the second reinforcing layer 150, but the number of reinforcing layers that the shaft 100 has may be one, three or more. The configuration of the reinforcing layer that the shaft 100 has can be changed as desired. For example, the shaft 100 may have one or more coil bodies and/or braids as reinforcing layers, or it may have a reinforcing layer other than the coil bodies and braids.

In the above embodiment, the distal-end-side third resin layer 131 or fourth resin layer 160 does not contain radiopaque material, but the distal-end-side third resin layer 131 or fourth resin layer 160 may contain radiopaque material at a lower content ratio of the radiopaque material than the content ratio of the radiopaque material of the distal tip 200.

The materials used to form the first resin layer 110, second resin layer 120, third resin layer 130, first reinforcing layer 140, second reinforcing layer 150, fourth resin layer 160, and distal tip 200 in the above embodiments are only examples, and each part may be formed of other materials.

In the above embodiment, the distal-end-side third resin layer 131 or fourth resin layer 160 and the distal tip 200 are different from each other in the property of radiopaque material content ratio, but instead of or in addition to this, other material property of both may be different from each other. The predetermined material property may be extensibility (Young's modulus). The predetermined material property may be breaking strength. The predetermined material property may be the type of resin material contained. The predetermined material property may be the grade (Shore hardness) of the resin material contained. The predetermined material property may be the type of radiopaque material contained. The catheter 10 may be inserted into a body lumen other than a blood vessel (for example, digestive organs such as bile duct, gallbladder, pancreas, esophagus, duodenum, small intestine, or large intestine, ureter, and trachea).

### [Reference Signs List]

10: Catheter 12: Lumen 100: Shaft 110: First resin layer 112: Distal end 120: Second resin layer 122: Distal end 130: Third resin layer 131: Distal-end-side third resin layer 132: Distal end 135: Proximal-end-side third resin layer 140: First reinforcing layer 141: Weld 142: Distal end 150: Second reinforcing layer 152: Distal end 160: Fourth resin layer 162: Distal end 200: Distal tip 202: Proximal end 210: Taper 220: Straight portion 300: Connector Ax: Center axis P1: Connection position

## Claims

1. A medical device comprising:
a distal tip containing resin material and radiopaque material;
a reinforcing layer connected to a proximal end of the distal tip; and
a resin layer arranged in a radially outward side of the distal tip, having a distal end located further in the distal end side than the proximal end of the distal tip, and having a predetermined material property different from the predetermined material property of the distal tip.

2. The medical device according to claim 1, wherein
the reinforcing layer is a first reinforcing layer,
the medical device further comprises a second reinforcing layer having a distal end located further in a distal end side than the proximal end of the distal tip, and
the distal end of the resin layer is located further in the distal end side than the distal end of the second reinforcing layer.

3. The medical device according to claim 2,
further comprising an inner layer arranged in a radially inward side of the second reinforcing layer and having a distal end located further in a distal end side than the distal end of the second reinforcing layer.

4. The medical device according to claim 2 or 3, wherein
the distal tip includes a taper that tapers toward a distal end and a straight portion arranged in a proximal end of the taper, and
the distal end of the second reinforcing layer is located further in the proximal end side than a boundary of the taper and the straight portion.

5. The medical device according to any one of claims 1 to 4,
wherein the predetermined material property is a content ratio of the radiopaque material, and the content ratio of the radiopaque material in the resin layer is lower than the content ratio of the radiopaque material in the distal tip.

6. The medical device according to any one of claims 1 to 5,
wherein the resin layer does not contain radiopaque material.

7. The medical device according to any one of claims 1 to 6,
wherein the resin layer and the distal tip contain the same type of resin material.
